# FASCICULE DE BREVET EUROPEEN

(11) **EP 2 828 657 B1**
(45) Date de publication et mention de la délivrance du brevet: **16.03.2016**
(21) Numéro de dépôt: 13719912.1
(22) Date de dépôt: 22.03.2013
(51) Int. Cl.: G01N 33/53, G01N 33/68, G01N 33/96, G01N 33/543

(54) **PROCÉDÉ DE DÉTERMINATION DE LA SUSCEPTIBILITÉ AUX INFECTIONS NOSOCOMIALES**
VERFAHREN ZUR BESTIMMUNG DER ANFÄLLIGKEIT FÜR NOSOKOMIALINFEKTIONEN
PROCESS FOR DETERMINING THE SUSCEPTIBILITY TO NOSOCOMIAL INFECTIONS

(30) Priorité: 23.03.2012 FR 1252641
(43) Date de publication de la demande: 28.01.2015
(73) Titulaire: Hospices Civils De Lyon, 69229 Lyon Cedex 02 (FR); Biomérieux, 69280 Marcy l'Étoile (FR)
(72) Inventeur: LEPAPE, Alain, F-69230 Saint-Genis-Laval (FR); VENET, Fabienne, F-01700 Beynost (FR); VILLARS, Astrid, 69680 Chassieu (FR); MONNERET, Guillaume, F-69003 Lyon (FR)
(74) Mandataire: Sarlin, Laure V.
(86) Numéro de dépôt international: PCT/FR2013/050624
(87) Numéro de publication internationale: WO 2013/140103

(56) Documents cités:
- WO-A1-2010/082004
- FR-A1- 2 941 240
- CHERON A ET AL: "Diminution de l'expression monocytaire de HLA-DR et risque d'infection hospitaliere", ANNALES FRANCAISES D'ANESTHESIE ET DE REANIMATION, MASSON, PARIS, FR, vol. 29, no. 5, 1 mai 2010 (2010-05-01), pages 368-376, XP027070333, ISSN: 0750-7658, DOI: 10.1016/J.ANNFAR.2010.02.015 [extrait le 2010-05-01]
- CAROLINE LANDELLE ET AL: "Low monocyte human leukocyte antigen-DR is independently associated with nosocomial infections after septic shock", INTENSIVE CARE MEDICINE, SPRINGER, BERLIN, DE, vol. 36, no. 11, 23 juillet 2010 (2010-07-23), pages 1859-1866, XP019836871, ISSN: 1432-1238
- FABIENNE VENET ET AL: "Clinical review: flow cytometry perspectives in the ICU - from diagnosis of infection to monitoring of injury-induced immune dysfunctions", CRITICAL CARE, vol. 15, no. 5, 1 janvier 2011 (2011-01-01), page 231, XP055049942, ISSN: 1364-8535, DOI: 10.1056/NEJMcibr1004371
- FABIENNE VENET ET AL: "Increased circulating regulatory T cells (CD4+CD25+CD127â ) contribute to lymphocyte anergy in septic shock patients", INTENSIVE CARE MEDICINE, SPRINGER, BERLIN, DE, vol. 35, no. 4, 23 octobre 2008 (2008-10-23), pages 678-686, XP019699367, ISSN: 1432-1238
- "Abstracts", INFLAMMATION RESEARCH ; OFFICIAL JOURNAL OF: THE INTERNATIONAL ASSOCIATION OF INFLAMMATION SOCIETIES THE EUROPEAN HISTAMINE RESEARCH SOCIETY, BIRKHÄUSER-VERLAG, BA, vol. 59, no. 1, 25 février 2010 (2010-02-25), pages 9-166, XP019792915, ISSN: 1420-908X

## Description

La présente invention concerne le domaine médical en général, et en particulier le domaine de la réanimation.

Plus précisément, l'invention concerne un procédé de détermination de la susceptibilité aux infections nosocomiales chez un patient hospitalisé en réanimation et/ou ayant subi une agression, telle que chirurgie, brûlure, traumatisme..., générant une réponse inflammatoire systémique ou SIRS, en particulier chez un patient en état septique, notamment sévère, et de préférence chez un patient en choc septique.

Les infections nosocomiales sont un problème de santé publique important. Les patients hospitalisés ont souvent, par nature, des défenses immunitaires diminuées ou altérées, du fait de pathologies portant directement atteinte à leurs compétences immunitaires, ou en raison de leur état général. Ces patients, et en particulier les personnes dénutries ou aux âges extrêmes de la vie (personnes âgées, nourrissons) sont spécialement sensibles aux infections en général, et en particulier à la survenue d'infections nosocomiales.

La prévalence des infections nosocomiales est nettement plus élevée dans les unités de réanimation que celle observée dans d'autres secteurs hospitaliers. L'incidence importante des infections nosocomiales dans ce secteur s'explique par la conjonction délétère de plusieurs facteurs de risque endogènes : une exposition du patient à des procédures invasives (ventilation artificielle, sondage urinaire, cathétérisme), la gravité des patients (ainsi que les comorbidités associées) et les thérapeutiques (transfusions multiples, sédation). Néanmoins malgré l'ensemble des mesures d'hygiène et de surveillance (risques exogènes) et la prise en compte de ces facteurs de risque endogènes, l'incidence des infections nosocomiales reste stable ou diminue modestement.

Déterminer la susceptibilité d'un patient aux infections nosocomiales est donc essentiel pour pouvoir proposer une prise en charge personnalisée, et ainsi tenter de minimiser les risques additionnels de décès.

A la connaissance des inventeurs, le seul marqueur immunologique actuellement connu pour être associé à une susceptibilité accrue aux infections nosocomiales est le marqueur HLA-DR (Human Leukocyte Antigen-DR) dont l'expression sur les monocytes (mHLA-DR) est diminuée chez les patients qui développent des infections nosocomiales. Cependant, l'expression de ce marqueur, développée en cytométrie en flux nécessite un équipement particulier (cytomètre en flux), qui est non disponible sur les plateaux de biologie (usage reservé à des laboratoires spécialisés d'hématologie ou d'immunologie) et dont la standardisation d'utilisation n'est pas établie. La mesure de mHLA-DR ne peut donc pas être réalisée en routine.

Les articles scientifiques suivants décrivent le marqueur HLA-DR comme étant associé à la susceptibilité aux infections nosocomiales chez un patient : Cheron A et al., "diminution de l'expression monocytaire de HLA-DR et risque d'infection hospitalière", Annales Françaises d'Anesthésie et de Réanimation 2010, 29(5) : 368-376 ;

Landelle C et al., "Low monocyte human leukocyte antigen-DR is independently associated with nosocomial infections after septic shock", Intensive Care Medicine 2010, 36(11): 1859-1866.

La susceptibilité aux infections nosocomiales peut aussi être déterminée par la mesure combinée de CD4, CD25 et CD127 à la surface des lymphoctes T : Venet F et al., "Clinical review: flow cytometry perspectives in the ICU - from diagnosis of infection to monitoring of injury-induced immune dysfunctions", Critical care 2011, 15(5):231;

Venet F et al., "Increased circulating regulatory T cells (CD4+CD25+CD127-) contribute to lymphocyte anergy in septic shock patients", Intensive Care Medicine 2008, 35(4):678-686.

Selon un premier aspect, la présente invention a donc pour objet un procédé de détermination de la susceptibilité aux infections nosocomiales chez un patient, comprenant les étapes suivantes :
- mesurer l'expression de sCD127 dans un échantillon biologique issu dudit patient ou échantillon à tester,
- conclure à une susceptibilité accrue aux infections nosocomiales après comparaison de l'expression de sCD127 par rapport à une valeur de référence.

Le procédé de détermination de la susceptibilité aux infections nosocomiales selon l'invention est donc un procédé mis en oeuvre *in vitro* ou *ex vivo.*

Le sCD127 est la forme soluble ou plasmatique du CD127, récepteur de l'IL-7. Le CD127 ou chaîne alpha du récepteur de l'IL-7 est une glycoprotéine de 75 kDa membre de la superfamille des récepteurs aux facteurs de croissance hématopoïétiques. Il est exprimé au niveau membranaire en association avec le CD132 (chaîne γ_{c} commune) pour former le récepteur de l'IL-7. Ce récepteur joue un rôle important dans la différenciation, la survie et la prolifération lymphocytaire. Le CD127 est constitué d'une partie extracellulaire de 219 acides aminés (aa), d'une partie transmembranaire de 25 aa et d'une partie intracytoplasmique de 195 aa. L'existence d'une forme soluble/plasmatique, nommée sCD127, générée par épissage alternatif de l'ARNm codant le CD127 a été décrite en 1990 par Goodwin RG et al., Cell, 1990, 23, 941-951, mais sa fonction biologique reste à ce jour mal connue.

On entend par « infection nosocomiale », toute infection, principalement bactérienne mais également virale et fongique, qui survient dans un établissement de santé au cours ou au décours d'une prise en charge (diagnostique, thérapeutique, palliative, préventive ou éducative), d'un patient, et qui n'était ni présente ni en incubation au début de la prise en charge. Lorsque l'état infectieux n'est pas connu avec précision au début de la prise en charge, un délai d'au moins 48 heures ou un délai supérieur à la période d'incubation est couramment accepté pour définir une infection nosocomiale.

Au sens de l'invention, on entend par « réponse inflammatoire systémique » ou « SIRS », une réponse associant au moins deux des critères suivants : Température > 38 °C ou < 36 °C, Fréquence cardiaque > 90/minute, Fréquence respiratoire > 20 / minute ou paCO2 <32 mmHg, Leucocytes > 12.000/mm3 ou < 4.000/mm3 (Bone et al., Chest, 1992, 1644-1655.

Dans le cadre de l'invention, on entend par « sCD127 » la forme soluble ou forme circulante (encore nommée forme plasmatique ou sérique) du récepteur de l'IL-7, encore nommé chaîne alpha du récepteur de l'IL-7 ou IL7R ou IL7R-ALPHA ou IL7RA ou CDW127, et en particulier telle que décrite par Goodwin et al, Cell, 1990, 23, 941-951 et dosée par Crawley et al, Journal of Immunology, 2010, 184, 4679-4687.

En particulier, les séquences nucléiques de référence pour sCD127 selon l'invention sont de préférence les suivantes : Ensembl : ENSG00000168685, HPRD-ID : 00893 Séquence nucléotidique : NM 002185.2, Vega genes : OTTHUMG00000090791.

Par ailleurs, les séquences protéiques de référence pour sCD127 selon l'invention sont de préférence les suivantes : NP_002176 XP_942460 ; version : NP_002176.2 GI:28610151.

L'échantillon à tester dans le cadre du procédé de l'invention est un échantillon biologique provenant du patient chez qui on souhaite déterminer la susceptibilité aux infections nosocomiales. En particulier, un tel échantillon biologique est choisi parmi ceux susceptibles de contenir le marqueur sCD127.

La présente invention présente une application particulièrement préférée chez les patients hospitalisés en réanimation et/ou ayant subi une agression, telle que chirurgie, brulure, traumatisme..., générant une réponse inflammatoire systémique ou SIRS. Par conséquent, l'échantillon biologique mis en oeuvre dans le cadre du procédé de l'invention est de préférence issu d'un patient hospitalisé en réanimation et/ou ayant subi une agression (chirurgie, brulure, traumatisme...) générant une réponse inflammatoire systémique (SIRS), en particulier issu d'un patient en état septique, notamment sévère, et en particulier issu d'un patient en choc septique. De manière particulièrement préférée, l'échantillon biologique mis en oeuvre dans le cadre du procédé de l'invention est issu d'un patient en choc septique.

Selon un premier mode de réalisation préféré, le procédé de l'invention permet de déterminer la susceptibilité d'un patient aux infections nosocomiales, c'est-à-dire conclure à un risque accru de développer une infection nosocomiale ou à une suceptibilité accrue aux infections nosocomiales, lorsqu'une surexpression de sCD127 est mise en évidence dans l'échantillon à tester par rapport à une première valeur de référence.

Par « surexpression », on entend une augmentation statistiquement significative du niveau d'expression.

Dans ce mode de réalisation, la première valeur de référence peut correspondre au niveau d'expression de sCD127 mesuré dans un échantillon biologique issu d'un patient hospitalisé en réanimation et/ou ayant subi une agression, telle que chirurgie, brulure, traumatisme..., générant une réponse inflammatoire systémique ou SIRS dont on sait qu'il n'a pas développé d'infection nosocomiale, notamment d'un patient en état septique dont on sait qu'il n'a pas développé d'infection nosocomiale, et de préférence d'un patient en choc septique dont on sait qu'il n'a pas développé d'infection nosocomiale. Dans ce cas, cette mesure de l'expression de sCD127 qui constitue la première valeur de référence est de préférence effectuée en parallèle, c'est-à-dire en même temps que la mesure de l'expression de sCD127 qui est faite dans l'échantillon issu du patient chez qui on cherche à déterminer la susceptibilité aux infections nosocomiales, bien que le prélèvement de l'échantillon de référence ait été effectuée antérieurement à celui de l'échantillon à tester.

Cette première valeur de référence peut également correspondre à une valeur moyenne du niveau d'expression de sCD127 qui est mesuré sur un pool d'échantillons issu de patients hospitalisés en réanimation et/ou ayant subi une agression, telle que chirurgie, brulure, traumatisme..., générant une réponse inflammatoire systémique (SIRS) dont on sait qu'ils n'ont pas développé d'infection nosocomiale, notamment de patients en état septique dont on sait qu'ils n'ont pas développé d'infection nosocomiale, et de préférence de patients en choc septique dont on sait qu'ils n'ont pas développé d'infection nosocomiale. Dans ce cas, cette mesure de l'expression de sCD127 qui constitue la première valeur de référence est de préférence effectuée préalablement à la mesure de l'expression de sCD127 qui est faite dans l'échantillon issu du patient chez qui on cherche à déterminer la susceptibilité aux infections nosocomiales, bien que le prélèvement des échantillons de référence destinés à être « poolés » ait été effectué antérieurement à celui de l'échantillon à tester.

Dans ce premier mode de réalisation préféré, et notamment pour déterminer la susceptiblité aux infections nosocomiales d'un patient en choc septique, la mesure de l'expression de sCD127 dans l'échantillon à tester et le cas échéant dans l'échantillon biologique utilisé pour obtenir la première valeur de référence, c'est-à-dire lorsque la première valeur de référence est obtenue à partir d'un échantillon biologique, est réalisée dans les 10 jours ou à 10 jours (J10) après le choc septique, de préférence dans les 7 jours ou à 7 jours (J7) après le choc septique, de manière encore préférée dans les 4 jours ou à 4 jours (J4) après le choc septique, et en particulier dans les 3 jours ou à 3 jours (J3) après le choc septique.

Selon un deuxième mode de réalisation préféré, le procédé de l'invention permet de déterminer la susceptibilité d'un patient aux infections nosocomiales, c'est-à-dire conclure à un risque accru de développer une infection nosocomiale ou à une susceptibilité accrue aux infections nosocomiales, lorsque l'expression de sCD127 qui est mesurée dans l'échantillon à tester n'est pas significativement diminuée par rapport à une deuxième valeur de référence. De manière particulièrement préférée, il est conclut à un risque accru de développer une infection nosocomiale si l'expression de sCD127 qui est mesurée dans l'échantillon à tester n'est pas diminuée de plus de 25 %, et en particulier pas diminuée de plus de 20 %, par rapport à cette deuxième valeur de référence.

Cette deuxième valeur de référence peut correspondre au niveau d'expression de sCD127 mesuré dans un échantillon biologique issu du même dit patient lors d'un prélèvement antérieur, c'est-à-dire dans un échantillon biologique qui a été prélevé antérieurement par rapport à l'échantillon à tester. Par « antérieurement » ou « antérieur », on entend de manière plus précoce dans le temps ou préalablement au prélèvement de l'échantillon à tester.

Dans ce deuxième mode de réalisation préféré, et notamment pour déterminer la susceptiblité aux infections nosocomiales d'un patient en choc septique, la mesure de l'expression de sCD127 dans l'échantillon à tester est réalisée environ ou à 10 jours (J10) après le choc septique, de préférence environ ou à 7 jours (J7) après le choc septique, de préférence encore environ ou à 4 jours (J4) après le choc septique.

Le prélèvement antérieur peut par exemple être effectué dans les ou à 48h après le choc septique et au moins 24h avant celui de l'échantillon à tester, et de préférence le prélèvement antérieur est effectué dans les ou à 48h après le choc septique et celui de l'échantillon à tester est effectué dans les 48h qui suivent le prélèvement antérieur ou à 48h après le prélèvement antérieur.

Ainsi, dans tous les cas, avant la mesure de l'expression de sCD127 proprement dite dans l'échantillon à tester, le procédé de l'invention peut comprendre l'obtention préalable de la valeur de référence, que ce soit la première valeur de référence ou la deuxième valeur de référence, à laquelle le niveau d'expression qui sera détecté dans l'échantillon à tester pourra être comparé afin de conclure à un risque accru ou non de développer des infections nosocomiales chez le patient duquel est issu l'échantillon à tester.

C'est donc à ces valeurs de référence, que ce soit la première valeur de référence ou la deuxième valeur de référence, obtenues préalablement ou en même temps, que sera comparée la valeur de l'expression de sCD127 qui est mesurée dans l'échantillon à tester.

L'échantillon sur lequel le procédé de l'invention est mis en oeuvre, encore nommé ici échantillon à tester, peut être d'origine animale ou humaine, et de préférence humaine.

Par conséquent, l'échantillon à tester peut être de différentes natures. En particulier, cet échantillon est un fluide biologique, par exemple choisi parmi le sang, le sang total (tel que collecté de la voie veineuse, c'est-à-dire contenant les cellules blanches et rouges, les plaquettes et le plasma), le sérum, le plasma et le liquide de lavage broncho-alvéolaire.

De préférence, l'échantillon à tester issu dudit patient est un échantillon de plasma ou de sérum.

Les échantillons à partir desquels peuvent être déterminées les valeurs de référence, que ce soit la première valeur de référence ou la deuxième valeur de référence, encore nommés « échantillons de référence », peuvent être de différentes natures et notamment de nature biologique tel que mentionné ci-dessus s'agissant de l'échantillon à tester (fluides biologiques). Avantageusement, ces échantillons biologiques sont de même nature que celle de l'échantillon biologique à tester ou tout du moins d'une nature compatible pour constituer une référence quant à la détection et/ou la quantification de l'expression de sCD127.

Pour obtenir la première valeur de référence en particulier, ces échantillons sont de préférence issus de personnes ayant les mêmes caractéristiques ou une majorité de caractéristiques communes, notamment du même sexe et/ou d'un âge similaire ou identique et/ou de même origine ethnique, avec celles du sujet ou patient chez qui on souhaite déterminer la susceptibilité aux infections nosocomiales. L'échantillon de référence peut également dans ce cas être constitué par tout échantillon, biologique ou non biologique, qui a été préalablement calibré pour contenir une valeur moyenne de sCD127 qui correspond au niveau qui a été mesuré sur un pool d'échantillons biologiques issus de patients, hospitalisés en réanimation et/ou ayant subi une agression, tell que chirurgie, brulure, traumatisme..., générant une réponse inflammatoire systémique (SIRS) dont on sait qu'ils n'ont pas développé d'infection nosocomiale, notamment de patients en état septique dont on sait qu'ils n'ont pas développé d'infection nosocomiale, et de préférence de patients en choc septique dont on sait qu'ils n'ont pas développé d'infection nosocomiale. Dans ce cas, et selon une variante particulièrement préférée, l'échantillon de référence est issu d'un ou de patients en choc septique dont on sait qu'il(s) n'a(ont) pas développé d'infection nosocomiale.

Pour obtenir la deuxième valeur de référence en particulier, l'échantillon de référence est un échantillon biologique issu du même dit patient, c'est-à-dire du patient chez qui on souhaite déterminer la susceptibilité aux infections nosocomiales et dont est issu l'échantillon à tester, mais obtenu à partir d'un prélèvement antérieur, c'est-à-dire d'un échantillon biologique qui a été prélevé antérieurement dans le temps par rapport à l'échantillon à tester.

Au sens de la présente invention, le terme « mesurer l'expression » s'entend être une mesure *in vitro* ou *ex vivo.* Par ailleurs, ce terme entend désigner la détection et la quantification de sCD127 au niveau protéique. A cet effet, toute méthode de détection et/ou de quantification bien connue de l'homme du métier peut être utilisée pour la mise en oeuvre de l'invention, que ce soit en rapport avec la détermination de la présence et/ou la mesure de l'expression de la protéine sCD127. A titre d'exemple de méthode de mesure de l'expression de la protéine sCD127, on peut notamment citer celle décrite par Crawley et al, Journal of Immuno/ogy, 2010, 184, 4679-4587.

En particulier, la mesure du niveau d'expression de sCD127 est réalisée à l'aide d'outils ou de réactifs qui sont spécifiques de sCD127 et qui permettent, directement ou indirectement, de déterminer sa présence et/ou de quantifier son niveau d'expression.

Parmi les outils ou réactifs capable(s) de détecter et/ou quantifier sCD127, on peut notamment citer des anticorps spécifiques, polyclonaux ou monoclonaux, de préférence monoclonaux, ou des fragments ou dérivés de ceux-ci, par exemple des anticorps « *single chain »* Sv.

Parmi ces outils ou réactifs, on préféra notamment ceux qui sont spécifiques de la forme soluble du récepteur de l'IL-7, c'est-à-dire qui ne reconnaissent pas CD127 qui est la forme cellulaire/membranaire non soluble de ce récepteur. On peut néanmoins utiliser des outils ou réactifs qui reconnaissent à la fois la forme soluble ou sCD127 et la forme cellulaire du récepteur de l'IL-7 ou CD127, tant qu'il est possible de distinguer ces deux formes par un autre moyen, comme par exemple la nature de l'échantillon analysé (par exemple, plasma ou sérum *versus* échantillon biologique contenant des cellules ou sang total).

Lorsque la détection et/ou la quantification de sCD127 est réalisée au niveau protéique, les techniques standards telles que le Western-Blot, ELISA, RIA, IRMA, FIA, CLIA, ECL, cytométrie de flux ou immunocytologie peuvent être utilisées.

De manière particulièrement avantageuse, l'expression de sCD127 est mesurée au niveau protéique, et de préférence à l'aide d'une technique ELISA.

Selon l'invention, et en particulier dans ce mode de réalisation particulier, le niveau d'expression de sCD127 est de préférence mesuré à l'aide d'anticorps anti-sCD127, monoclonaux ou polyclonaux, et notamment d'anticorps monoclonaux anti-sCD127. A titre d'exemple, on peut notamment citer les anticorps monoclonaux anti-CD127 humain R34.34 commercialisés par la société Beckman Coulter® ou les anticorps polyclonaux anti-CD127 commercialisés par la société R&D Systems®.

Toutes les indications et préférences mentionnées ci-dessus s'agissant de la mesure de l'expression de sCD127 s'appliquent indifféremment que ce soit pour la mesure de cette expression dans l'échantillon à tester et dans l'échantillon de référence.

Selon un deuxième aspect, la présente invention a également pour objet l'utilisation de la mesure, *in vitro* ou *ex vivo,* de l'expression de sCD127 pour déterminer la susceptibilité d'un patient aux infections nosocomiales.

De manière préférée, cette utilisation est particulièrement avantageuse pour déterminer la susceptibilité aux infections nosocomiales d'un patient hospitalisé en réanimation et/ou ayant subi une agression, telle que chirurgie, brulure, traumatisme..., générant une réponse inflammatoire systémique ou SIRS, en particulier d'un patient en état septique, notamment sévère, et de préférence d'un patient en choc septique. De préférence, l'utilisation selon l'invention permet de déterminer la susceptibilité aux infections nosocomiales d'un patient en choc septique.

Par ailleurs, dans le cadre de l'utilisation selon l'invention, l'expression de sCD127 est de préférence mesurée au niveau protéique, et en particulier à l'aide d'une technique ELISA.

En particulier, l'expression de sCD127 peut être mesurée à l'aide d'anticorps anti-sCD127, monoclonaux ou polyclonaux, et de préférence d'anticorps monoclonaux anti-sCD127. Les anticorps cités ci-dessus peuvent également être utilisés pour ce deuxième aspect de l'invention.

Plus largement, tous les modes de réalisation préférés qui sont mentionnés ci-dessus concernant le procédé et leurs combinaisons constituent également des modes de réalisation préférés s'agissant de l'utilisation.

Selon un troisième aspect, la présente invention a aussi pour objet un kit pour la mesure, *in vitro* ou *ex vivo,* de l'expression de sCD127 dans un échantillon biologique, comprenant :
- des outils ou réactifs spécifiques permettant de mesurer l'expression de sCD127 dans ledit échantillon biologique, et
- un échantillon contrôle positif qui est un échantillon calibré pour contenir la quantité de sCD127 qui correspond à la quantité moyenne mesurée dans un pool d'échantillons de patients dont on sait qu'ils ont développé une infection nosocomiale, et/ou un échantillon contrôle négatif qui est un échantillon calibré pour contenir la quantité de sCD127 qui correspond à la quantité moyenne mesurée dans un pool d'échantillons de patients dont on sait qu'ils n'ont pas développé d'infection nosocomiale.

Ainsi, le kit selon l'invention comprend des outils ou réactifs spécifiques permettant de mesurer l'expression de sCD127 dans ledit échantillon biologique, et au moins un échantillon contrôle.

Le kit selon l'invention permet en particulier de déterminer la susceptibilité aux infections nosocomiales chez un patient, et notamment chez un patient en choc septique.

De préférence, les outils ou réactifs spécifiques permettant de mesurer l'expression de sCD127 dans un échantillon biologique qui sont présents dans le kit de l'invention permettent de détecter et/ou de quantifier l'expression de sCD127, que ce soit au niveau protéique ou au niveau de l'activité de sCD127, et de préférence au niveau protéique.

Selon un mode de réalisation particulièrement préféré, le kit de l'invention contient des anticorps anti-sCD127, monoclonaux ou polyclonaux, et en particulier des anticorps monoclonaux.

Un autre échantillon contrôle positif peut également être un échantillon issu d'un patient dont on sait qu'il a développé une infection nosocomiale. De même, un autre échantillon contrôle négatif peut également être un échantillon issu d'un patient dont on sait qu'il n'a pas développé d'infection nosocomiale. Que ce soit pour un contrôle positif ou négatif ce type d'échantillon contrôle est de préférence issu d'un ou de plusieurs patient(s) hospitalisé(s) en réanimation et/ou ayant subi une agression, telle que chirurgie, brulure, traumatisme..., générant une réponse inflammatoire systémique (SIRS), notamment d'un ou de plusieurs patient(s) en état septique, et de préférence d'un ou de plusieurs patient(s) en choc septique. Par exemple, le kit peut contenir un échantillon contrôle négatif issu d'un ou de plusieurs patient(s) hospitalisé(s) en réanimation et/ou ayant subi une agression, telle que chirurgie, brulure, traumatisme..., générant une réponse inflammatoire systémique (SIRS), dont on sait qu'il(s) n'a(ont) pas développé d'infection nosocomiale, en particulier issu d'un ou de plusieurs patient(s) en choc septique dont on sait qu'il(s) n'a(ont) pas développé d'infection nosocomiale.

De manière préférée, le kit comprend à la fois un échantillon contrôle positif et un échantillon contrôle négatif, et en particulier choisis chacun parmi les échantillons calibrés tels que définis ci-dessus.

L'invention couvre également l'utilisation d'un kit selon l'invention pour réaliser le procédé de l'invention, et en particulier pour déterminer la susceptibilité d'un patient aux infections nosocomiales, de préférence chez un patient hospitalisé en réanimation et/ou ayant subi une agression, telle que chirurgie, brulure, traumatisme..., générant une réponse inflammatoire systémique (SIRS), en particulier chez un patient en état septique, notamment sévère, et de préférence un patient en choc septique. De préférence, l'utilisation du kit selon l'invention permet de déterminer la susceptibilité aux infections nosocomiales chez un patient en choc septique.

Tous les modes de réalisation préférés qui sont mentionnés ci-dessus concernant le procédé et leurs combinaisons constituent également des modes de réalisation préférés s'agissant du kit selon l'invention et de son utilisation.

Diverses autres caractéristiques ressortent de la description faite ci-dessous en référence aux figures annexées qui montrent, à titre d'exemples non limitatifs, des formes de réalisation de l'objet de l'invention et dans lesquelles :
- la **figure 1** représente la concentration d'IL-7 plasmatique chez 35 patients en choc septique aux jours 1-2 (1-2) et 3-4 (3-4) et 30 sujets sains « HV » (A) et la comparaison de ces résultats selon les groupes de patients « NS » ou « S » (figure 1B) et les groupes de patients « NI » ou « No NI » (figure 1C).
   ** p<0,005 vs. « HV » - U-test Mann Whitney ;
- la **figure 2** représente l'expression de CD127 sur les cellules T CD4⁺ (figure 2A) et sur les cellules T CD8⁺ (figure 2B) chez 35 patients en choc septique aux jours 1-2 et 3-5 et 30 sujets sains « HV ».
   * p<0,05 vs « HV » - U-test Mann Whitney ; § p<0,05 - évolution dans le temps dans un même groupe de patients - *Wilcoxon paired t-test.*
- la **figure 3** représente la concentration de sCD127 plasmatique chez 35 patients en choc septique aux jours 1-2 et 3-4 et 30 sujets sains (figure 3A) et la comparaison de ces résultats selon les groupes de patients « NS » ou « S » (figure 3B) et les groupes de patients « NI » ou « No NI » (figure 3C).
   # p<0,05 ## p<0,005 vs « No NI » - U-test Mann Whitney ; § p<0,05 §§ p<0,005 - évolution dans le temps dans un même groupe de patients - *Wilcoxon paired t-test.*
- la **figure 4** représente l'expression du marqueur HLA-DR chez 35 patients en choc septique aux jours 1-2 et 3-4 et 30 sujets sains selon les groupes de patients « NI » ou « No NI ».

### METHODES

### Mesure de la concentration en IL-7 plasmatigue

La concentration d'IL-7 plasmatique a été mesurée grâce à un kit utilisant la technique LUMINEX™ commercialisé par la société Bio-Rad (BioPlex Pro Cytokine, Chemokine and Growth Factor Assays : BioPlex Pro Reagent kit, Bio-Rad #171-304070 et SinglePlex IL-7) en suivant les instructions du fournisseur.

### Mesure de l'expression cellulaire du récepteur de l'IL-7 (CD127)

Brièvement, 50µl de sang total est incubé en présence de 5µl d'Ac anti-CD4 couplé Phycoérythine-TexasRed (ECD) (BeckmanCoulter #6604727) ou de 5µl d'Ac anti-CD8 couplé ECD (BeckmanCoulter #737659) ainsi que 10µl d'Ac anti-CD127 couplé à la phycoérythrine (PE) (BeckmanCoulter #IM1980U) pendant 15 minutes à température ambiante et à l'obscurité. Les globules rouges sont ensuite lysés grâce à une lyse hypotonique et les cellules fixées grâce à une lyse automatique réalisée sur un automate TQ-Prep (BeckmanCoulter). L'expression membranaire du CD127 à la surface des lymphocytes T CD4+ ou CD8+ est enfin mesurée par cytométrie en flux. ***Dosage de la forme soludle du récepteur de l'IL***-***7 (sCD127) par ELISA***

### « Coating »

Un tampon de « coating » a été préparé pour contenir 0,8 g Na₂CO₃, 1,4 g NaHCO₃, 0,1 g NaN₃ dans 500 ml d'eau (pH 9.6).

100 µL d'anticorps (Ac) de capture (anticorps monoclonal de souris anti-CD127 humain R34.34, Beckman Coulter®) dilués dans du tampon de « coating » (ont été déposés par puits dans une plaque ([Ac] = 8 µg/mL). La plaque a ensuite été recouverte pour être incubée à 4°C pendant une nuit.

Le contenu des puits a ensuite été aspiré et les puits ont été lavés 3 fois avec au moins 300µL de tampon de lavage PBS-Tween₂₀ 0,05%. Tout le liquide a soigneusement été enlevé à chaque lavage. Après le dernier lavage, la plaque a été retournée sur du papier absorbant afin d'éliminer toutes les traces de tampon.

### « Blocking »

La fixation non spécifique a été bloquée à l'aide de 150 µL de tampon de blocage par puits (10% sérum de bovin foetal (FBS)/PBS-Tween₂₀ 0,05%), puis la plaque a été incubée pendant 1h à 37°C.

De nouveau, le contenu des puits a été aspiré et les puits ont été lavés 3 fois avec au moins 300µL de tampon de lavage PBS-Tween₂₀ 0,05%. Tout le liquide a soigneusement été enlevé à chaque lavage. Après le dernier lavage, la plaque a été retournée sur du papier absorbant afin d'éliminer toutes les traces de tampon.

### Échantillons et contrôles

Une gamme étalon a été réalisée avec recombinant human IL-7Rα / CD127 Fc chimera (R&D Systems - Catalog Number: 306-IR) dilué dans du tampon de dilution PBS 5 % FCS, comme décrit dans le tableau 1 ci-dessous et conformément à C. Janot-Sardet et al. Journal of Immunological Methods, 2010, 28, 115-123.

**Tableau 1**

| | rh IL-7Rα / CD127 Fc chimera | | | | | | | |
|---|---|---|---|---|---|---|---|---|
| [c] (ng/mL) | 500 | 250 | 125 | 62.5 | 31.25 | 15.7 | 7.85 | 0 |
| Diluant (µL) | 0 | 100 | 100 | 100 | 100 | 100 | 100 | 100 |
| Solution à 500 ng/mL (µL) | 100 | 100 | Dilutions successives | | | | | 0 |

100 µL d'échantillon ou de contrôle (solution de CD127 Fc chimera reconstitué extemporanément et aliquoté à des concentrations de 60ng/ml et 10ng/ml) ont été ajoutés dans chaque puits, puis la plaque a été incubée pendant 1h à 37°C.

De nouveau, le contenu des puits a été aspiré et les puits ont été lavés 3 fois avec au moins 300µL de tampon de lavage PBS-Tween₂₀ 0,05%. Tout le liquide a soigneusement été enlevé à chaque lavage. Après le dernier lavage, la plaque a été retournée sur du papier absorbant afin d'éliminer toutes les traces de tampon.

### Anticorps de détection

100 µL d'anticorps de détection (anticorps de chèvre polyclonal anti-CD127 biotinylé reconstitués avec 1 mL de TBS-BSA 1%, R&D Systems®) dilués dans du PBS/5 %FBS ont été ajoutés dans chaque puits ([Ac] = 200 ng/mL), puis la plaque a été incubée pendant 1h à 37°C.

A nouveau, le contenu des puits a été aspiré et les puits ont été lavés 3 fois avec au moins 300µL de tampon de lavage PBS-Tween₂₀ 0,05%. Tout le liquide a soigneusement été enlevé à chaque lavage. Après le dernier lavage, la plaque a été retournée sur du papier absorbant afin d'éliminer toutes les traces de tampon.

### Révélation

100 µL de Streptavidin-HRP ont été ajoutés dans chaque puits ([Streptavidin-HRP] = 8 µL/mL). La plaque a ensuite été recouverte pour être incubée pendant 30 min à température ambiante.

A nouveau, le contenu des puits a été aspiré et les puits ont été lavés 3 fois avec au moins 300µL de tampon de lavage PBS-Tween₂₀ 0,05%. Tout le liquide a soigneusement été enlevé à chaque lavage. Après le dernier lavage, la plaque a été retournée sur du papier absorbant afin d'éliminer toutes les traces de tampon. A cette étape de lavage, les puits ont été imbibés avec le tampon de lavage 1 à 2 min avant aspiration.

Les deux flacons de la solution de substrat colorimétrique TMB (3,3',5,5'-tetramethylbenzidine, bioMérieux #XX7LF1UC) ont été mélangés volume à volume. 100 µL de cette solution de substrat ont été déposés dans chaque puits, La plaque a ensuite été recouverte pour être incubée pendant 30 min à température ambiante.

Enfin, la lecture de la plaque a été effectuée par mesure de l'absorbance à 450 nm.

### Mesure de l'expression monocytaire de HLA-DR

La mesure a été réalisée par la technique de cytométrie en flux, à l'aide d'un marquage direct en sang total prélevé sur EDTA.

Les deux anticorps suivants ont été incubés avec 50 µL de sang total (30 minutes à température ambiante et à l'obscurité) :
- 5 µL d'anticorps anti-CD14, couplés fluoresceine (Beckman Coulter Immunotech, - Réf. : IM0645), permettant d'identifier les monocytes parmi les leucocytes)
- 10 µL d'anticorps anti-HLA-DR, couplés phycoérythrine (Becton Dickinson - Réf. : 347401), permettant de quantifier l'expression de HLA-DR à la surface des cellules.

Les globules rouges ont ensuite été éliminés : lyse par ajout de 1 ml de solution de lyse (commercialisée par la société Becton Dickinson sous la référence 349202) diluée (1/10) (15 minutes à température ambiante et à l'obscurité).

Les cellules ont ensuite été analysées sur un cytométre de flux (FC500 - Beckman Coulter).

Les résultats sont exprimés en % de cellules positives, le seuil de positivité étant défini grâce à un contrôle isotypique (Mouse IgG2a PE Becton Dickinson - Réf. : 349053).

### RESULTATS

Des échantillons de plasma ont été prélevés sur 35 patients en choc septique aux jours 1-2 (J1-2) et 3-4 (J3-4) après choc septique, puis ont été stockés (cohorte rétrospective). Différents paramètres ou marqueurs ont été mesurés tels que les concentrations d'IL-7 et de sCD127 plasmatiques, et l'expression de CD127 sur les cellules T CD4⁺, et l'expression de HLA-DR sur les monocytes. A 28 jours après admission en réanimation pour choc septique, 13 patients n'ont pas survécu (« NS ») soit 37 % alors que 22 patients ont survécu (« S ») sur les 35 patients. Par ailleurs, 6 patients ont contracté une infection nosocomiale (« NI ») soit 17 %, alors que 29 patients sont restés exempt de toute épisode nosocomiale (« No NI »).

Les mêmes mesures ont également été effectuées sur 30 sujets sains volontaires (HV).

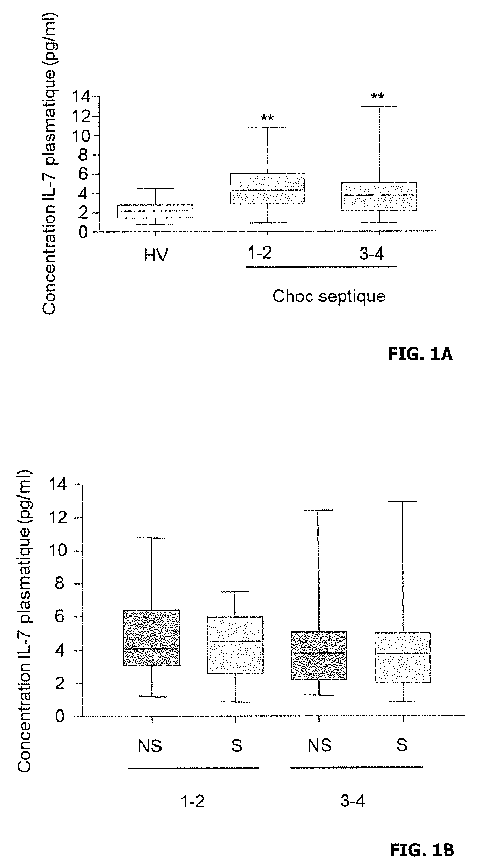

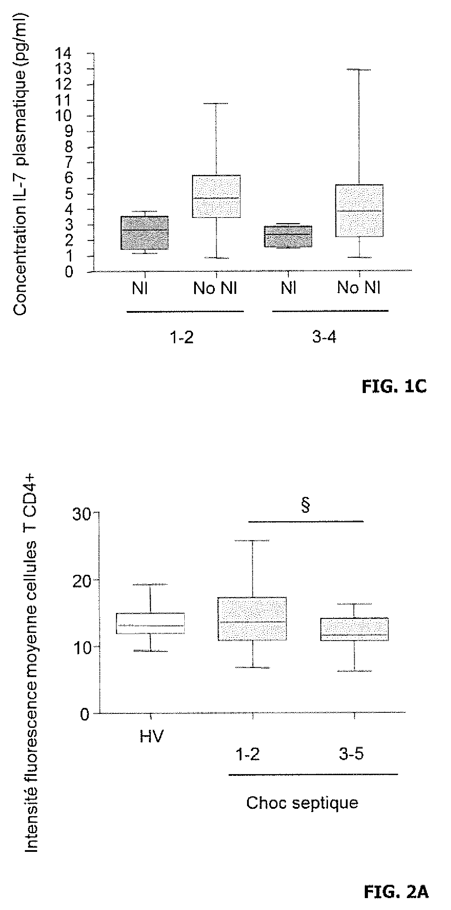

Les résultats obtenus ont été comparés à l'aide d'un test U Mann Whitney au sein de ces différentes populations de sujets ou patients et sont regroupés sur les figures 1 à 4 annexées.

Ces résultats montrent que la concentration d'IL-7 plasmatique est significativement diminuée (à J1-2, mais ce n'est pas significatif à J3-4 chez les patients qui développent une infection secondaire par rapport aux patients qui n'ont rencontré aucun épisode nosocomial (figure 1C), alors qu'elle reste inchangée entre les patients survivants « S » ou non-survivants « NS ».

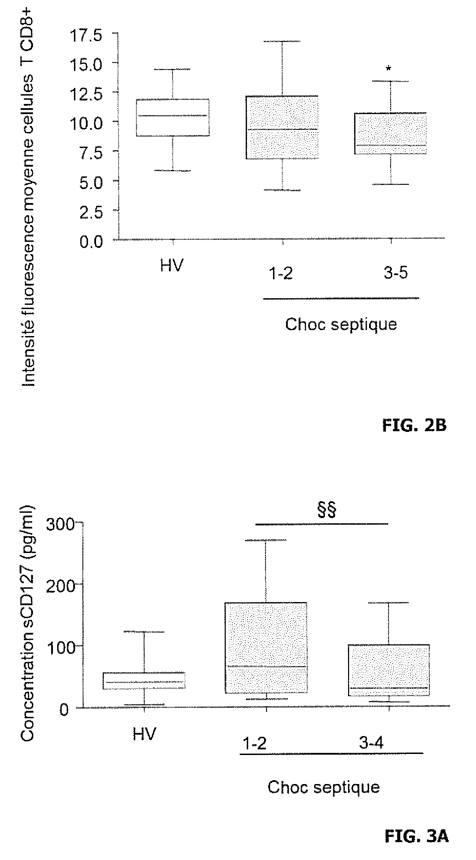

De plus, l'expression cellulaire du récepteur de l'IL-7 (CD127) est conservée après choc septique (figure 2A et 2B), et sans aucune différence entre les patients survivants « S » ou non-survivants « NS » ou les patients qui ont développé une infections nosocomiale « NI » ou pas « No NI » (résultats non représentés).

La légère augmentation de la concentration plasmatique de la forme soluble du récepteur de l'IL-7 ou sCD127 observée au début du choc septique n'est pas statistiquement significative et revient à des valeurs « normales » avec le temps (figure 3A).

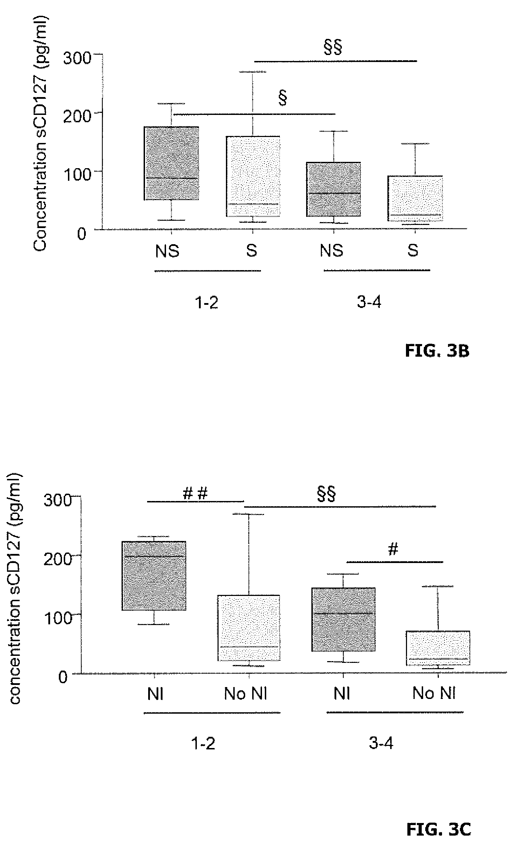

En revanche, alors que la concentration plasmatique de sCD127 reste inchangée entre les patients survivants « S » ou non-survivants « NS » (figure 3B), on observe une augmentation marquée de la concentration plasmatique de sCD127 chez les patients « NI » qui ont présenté une infection nosocomiale par rapport aux patients « No NI » qui n'ont pas contracté d'infection nosocomiale (figure 3C).

Par ailleurs, on observe également que la concentration plasmatique du sCD127 n'évolue pas dans le temps (entre J1-2 et J3-4) chez les patients « NI » qui ont présenté une infection nosocomiale, contrairement aux patients « No NI » qui n'ont pas contracté d'infection nosocomiale (figure 3C).

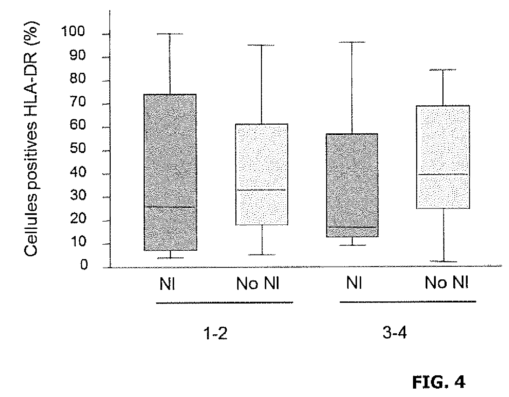

A titre de comparaison, l'étude de l'expression du marqueur de l'art antérieur HLA-DR sur la même cohorte de patients n'a pas permis de mettre en évidence la diminution de l'expression de ce marqueur entre les patients « NI » qui ont présenté une infection nosocomiale et les patients « No NI » qui n'ont pas contracté d'infection nosocomiale (figure 4), contrairement à ce qui est clairement établi dans l'art antérieur.

Cette différence, probablement liée à la taille de la cohorte présentement étudiée qui est plus petite par rapport à celle des cohortes analysées dans les études de l'art antérieur, permet de mettre en évidence la pertinence du marqueur sCD127 par rapport à HLA-DR. En effet, malgré la taille de la cohorte qui a été étudiée ici, les résultats montrent que l'étude de l'expression de sCD127 selon l'invention est informative quant à la susceptibilité des patients aux infections nosocomiales.

En conséquence, l'ensemble de ces résultats démontrent que la mesure de l'expression de sCD127 est un marqueur immunologique utile pour déterminer la susceptibilité d'un patient aux infections nosocomiales, et en particulier des patients hospitalisés en réanimation et/ou ayant subi une agression (chirurgie, brulure, traumatisme...) générant une réponse inflammatoire systémique (SIRS), et en particulier des patients en état septique, notamment sévère, et de préférence des patients en choc septique.

L'invention n'est pas limitée aux exemples décrits et représentés car diverses modifications peuvent y être apportées sans sortir de son cadre.

## Revendications

1. Procédé de détermination de la susceptibilité aux infections nosocomiales chez un patient, comprenant les étapes suivantes :
- mesurer l'expression de sCD127 dans un échantillon biologique issu dudit patient ou échantillon à tester,
- conclure à une susceptibilité accrue aux infections nosocomiales après comparaison de l'expression de sCD127 par rapport à une valeur de référence.

2. Procédé selon la revendication 1, **caractérisé en ce que** l'échantillon biologique est issu d'un patient hospitalisé en réanimation et/ou ayant subi une agression, telle que chirurgie, brulure, traumatisme..., générant une réponse inflammatoire systémique ou SIRS, en particulier issu d'un patient en état septique, notamment sévère.

3. Procédé selon la revendication 2, **caractérisé en ce que** l'échantillon biologique est issu d'un patient en choc septique.

4. Procédé selon l'une quelconque des revendications 1 à 3, **caractérisé en ce qu'**il est conclut à une susceptibilité accrue aux infections nosocomiales lorsqu'une surexpression de sCD127 est mise en évidence dans l'échantillon à tester par rapport à une première valeur de référence et **en ce que** la première valeur de référence correspond au niveau d'expression de sCD127 mesuré dans un échantillon biologique issu d'un patient hospitalisé en réanimation et/ou ayant subi une agression, telle que chirurgie, brûlure ou traumatisme, générant une réponse inflammatoire systémique ou SIRS dont on sait qu'il n'a pas développé d'infection nosocomiale, notamment d'un patient en état septique dont on sait qu'il n'a pas développé d'infection nosocomiale, ou bien correspond à une valeur moyenne du niveau d'expression de sCD127 qui est mesuré sur un pool d'échantillons issu de tels patients.

5. Procédé selon la revendication 4, **caractérisé en ce que** la première valeur de référence correspond au niveau d'expression de sCD127 mesuré dans un échantillon biologique issu d'un patient en choc septique dont on sait qu'il n'a pas développé d'infection nosocomiale, ou bien correspond à une valeur moyenne du niveau d'expression de sCD127 qui est mesuré sur un pool d'échantillons issu de tels patients.

6. Procédé selon l'une quelconque des revendications 3 à 5, **caractérisé en ce que** la mesure de l'expression de sCD127 dans l'échantillon à tester et le cas échéant dans l'échantillon biologique utilisé pour obtenir la première valeur de référence est réalisée dans les 10 jours ou à 10 jours (J10) après le choc septique, de préférence dans les 7 jours ou à 7 jours (J7) après le choc septique, de manière encore préférée dans les 4 jours ou à 4 jours (J4) après le choc septique, et en particulier dans les 3 jours ou à 3 jours (J3) après le choc septique.

7. Procédé selon l'une quelconque des revendications 1 à 3, **caractérisé en ce qu'**il est conclut à une susceptibilité accrue aux infections nosocomiales lorsque l'expression de sCD127 qui est mesurée dans l'échantillon à tester n'est pas significativement diminuée par rapport à une deuxième valeur de référence, de préférence lorsque l'expression de sCD127 qui est mesurée dans l'échantillon à tester n'est pas diminuée de plus de 25 %, et de préférence pas diminuée de plus de 20 %, par rapport à cette deuxième valeur de référence et **en ce que** la deuxième valeur de référence correspond au niveau d'expression de sCD127 mesuré dans un échantillon biologique issu du même dit patient lors d'un prélèvement antérieur, c'est-à-dire dans un échantillon biologique qui a été prélevé antérieurement par rapport à l'échantillon à tester.

8. Procédé selon la revendication 3 ou 7, **caractérisé en ce que** la mesure de l'expression de sCD127 dans l'échantillon à tester est réalisée à environ ou 10 jours (J10) après le choc septique, de préférence à environ ou 7 jours (J7) après le choc septique, de préférence encore à environ ou 4 jours (J4) après le choc septique.

9. Procédé selon la revendication 8, **caractérisé en ce qu'**il est conforme à la revendication 7 et **en ce que** le prélèvement antérieur est effectué dans les ou à 48h après le choc septique et au moins 24h avant celui de l'échantillon à tester.

10. Procédé selon la revendication 9, **caractérisé en ce que** le prélèvement antérieur est effectué dans les ou à 48h après le choc septique et celui de l'échantillon à tester est effectué dans les 48h qui suivent le prélèvement antérieur ou à 48h après le prélèvement antérieur.

11. Procédé selon l'une quelconque des revendications précédentes, **caractérisé en ce que** l'échantillon à tester issu dudit patient est un échantillon de plasma ou de sérum.

12. Procédé selon l'une quelconque des revendications précédentes, **caractérisé en ce que** l'expression de sCD127 est mesurée au niveau protéique, de préférence à l'aide d'une technique ELISA.

13. Procédé selon l'une quelconque des revendications précédentes, **caractérisé en ce que** l'expression de sCD127 est mesurée à l'aide d'anticorps anti-sCD127, et notamment d'anticorps monoclonaux anti-sCD127.

14. Utilisation de la mesure, *in vitro* ou *ex vivo,* de l'expression de sCD127 pour déterminer la susceptibilité d'un patient aux infections nosocomiales.

15. Utilisation selon la revendication 14, **caractérisée en ce que** ledit patient est un patient hospitalisé en réanimation et/ou ayant subi une agression, telle que chirurgie, brûlure ou traumatisme, générant une réponse inflammatoire systémique (SIRS), en particulier d'un patient en état septique, notamment sévère.

16. Utilisation selon la revendication 15, **caractérisée en ce que** ledit patient est un patient en choc septique.

17. Utilisation selon la revendication 15 ou 16, **caractérisée en ce que** l'expression de sCD127 est mesurée au niveau protéique, de préférence à l'aide d'une technique ELISA.

18. Utilisation selon l'une quelconque des revendications 14 à 17, **caractérisée en ce que** l'expression de sCD127 est mesurée à l'aide d'anticorps anti-sCD127, de préférence d'anticorps monoclonaux anti-sCD127.

19. Kit pour la mesure, *in vitro* ou *ex vivo,* de l'expression de sCD127 dans un échantillon biologique, comprenant :
- des outils ou réactifs spécifiques permettant de mesurer l'expression de sCD127 dans ledit échantillon biologique, et
- un échantillon contrôle positif qui est un échantillon calibré pour contenir la quantité de sCD127 qui correspond à la quantité moyenne mesurée dans un pool d'échantillons de patients dont on sait qu'ils ont développé une infection nosocomiale, et/ou un échantillon contrôle négatif qui est un échantillon calibré pour contenir la quantité de sCD127 qui correspond à la quantité moyenne mesurée dans un pool d'échantillons de patients dont on sait qu'ils n'ont pas développé d'infection nosocomiale.

20. Kit selon la revendication 19, **caractérisé en ce que** lesdits outils ou réactifs spécifiques permettent de détecter et/ou de quantifier l'expression de sCD127, et de préférence au niveau protéique.

21. Utilisation d'un kit tel que défini à la revendication 19 ou 20 pour déterminer la susceptibilité d'un patient aux infections nosocomiales, ledit patient étant un patient hospitalisé en réanimation et/ou ayant subi une agression, telle que chirurgie, brûlure ou traumatisme, générant une réponse inflammatoire systémique (SIRS).

22. Utilisation d'un kit selon la revendication 21 pour déterminer la susceptibilité aux infections nosocomiales chez un patient en choc septique.

## Patentansprüche

1. Verfahren zur Bestimmung der Anfälligkeit für nosokomiale Infektionen bei einem Patienten, umfassend die folgenden Schritte:
- Messen der Expression von sCD127 in einer von dem Patienten stammenden biologischen Probe oder zu testenden Probe,
- Schließen auf eine erhöhte Anfälligkeit für nosokomiale Infektionen nach Vergleich der Expression von sCD127 mit einem Referenzwert.

2. Verfahren nach Anspruch 1, **dadurch gekennzeichnet, dass** die biologische Probe von einem Patienten stammt, der auf der Intensivstation aufgenommen ist und/oder der einen Stress, wie chirurgischen Eingriff, Verbrennung, Trauma etc., der eine systemische Inflammationsreaktion oder SIRS erzeugt, erfahren hat, insbesondere von einem Patienten in insbesondere schwerem septischem Zustand stammt.

3. Verfahren nach Anspruch 2, **dadurch gekennzeichnet, dass** die biologische Probe von einem Patienten in septischem Schock stammt.

4. Verfahren nach einem der Ansprüche 1 bis 3, **dadurch gekennzeichnet, dass** auf eine erhöhte Anfälligkeit für nosokomiale Infektionen geschlossen wird, wenn eine Überexpression von sCD127 in der zu testenden Probe gegenüber einem ersten Referenzwert nachgewiesen wird und dass der erste Referenzwert dem Expressionsniveau von sCD127 entspricht, das in einer biologischen Probe gemessen wird, die von einem Patienten stammt, der auf der Intensivstation aufgenommen ist und/oder der einen Stress, wie chirurgischen Eingriff, Verbrennung oder Trauma, der eine systemische Inflammationsreaktion oder SIRS erzeugt, erfahren hat und von dem man weiß, dass er keine nosokomiale Infektion entwickelt hat, insbesondere von einem Patienten in septischem Zustand, von dem man weiß, dass er keine nosokomiale Infektion entwickelt hat, bzw. einem Durchschnittswert des Expressionsniveaus von sCD127 entspricht, das an einem von solchen Patienten stammenden Probenpool gemessen wird.

5. Verfahren nach Anspruch 4, **dadurch gekennzeichnet, dass** der erste Referenzwert dem Expressionsniveau von sCD127 entspricht, das in einer biologischen Probe gemessen wird, die von einem Patienten in septischem Schock stammt, von dem man weiß, dass er keine nosokomiale Infektion entwickelt hat, bzw. einem Durchschnittswert des Expressionsniveaus von sCD127 entspricht, das an einem von solchen Patienten stammenden Probenpool gemessen wird.

6. Verfahren nach einem der Ansprüche 3 bis 5, **dadurch gekennzeichnet, dass** die Messung der Expression von sCD127 in der zu testenden Probe und gegebenenfalls in der biologischen Probe, die für den Erhalt des ersten Referenzwertes verwendet wird, innerhalb von 10 Tagen oder 10 Tage (J10) nach dem septischen Schock, vorzugsweise innerhalb von 7 Tagen oder 7 Tage (J7) nach dem septischen Schock, weiterhin bevorzugt innerhalb von 4 Tagen oder 4 Tage (J4) nach dem septischen Schock und insbesondere innerhalb von 3 Tagen oder 3 Tage (J3) nach dem septischen Schock durchgeführt wird.

7. Verfahren nach einem der Ansprüche 1 bis 3, **dadurch gekennzeichnet, dass** auf eine erhöhte Anfälligkeit für nosokomiale Infektionen geschlossen wird, wenn die Expression von sCD127, die in der zu testenden Probe gemessen wird, gegenüber einem zweiten Referenzwert nicht signifikant abgenommen hat, vorzugsweise wenn die Expression von sCD127, die in der zu testenden Probe gemessen wird, nicht um mehr als 25 % abgenommen hat und vorzugsweise nicht um mehr als 20 % gegenüber diesem zweiten Referenzwert abgenommen hat, und dass der zweite Referenzwert dem Expressionsniveau von sCD127 entspricht, das in einer biologischen Probe, welche von dem gleichen Patienten aus einer früheren Entnahme stammt, das heißt in einer biologischen Probe, die früher als die zu testende Probe entnommen worden ist, gemessen wird.

8. Verfahren nach Anspruch 3 oder 7, **dadurch gekennzeichnet, dass** die Messung der Expression von sCD127 in der zu testenden Probe etwa bei oder 10 Tage(n) (J10) nach dem septischen Schock, vorzugsweise etwa bei oder 7 Tage(n) (J7) nach dem septischen Schock, weiterhin vorzugsweise etwa bei oder 4 Tage(n) (J4) nach dem septischen Schock durchgeführt wird.

9. Verfahren nach Anspruch 8, **dadurch gekennzeichnet, dass** es gemäß Anspruch 7 ist und dass die frühere Entnahme innerhalb von oder bei 48h nach dem septischen Schock und wenigstens 24h vor derjenigen der zu testenden Probe durchgeführt wird.

10. Verfahren nach Anspruch 9, **dadurch gekennzeichnet, dass** die frühere Entnahme innerhalb von oder bei 48h nach dem septischen Schock durchgeführt wird und diejenige der zu testenden Probe innerhalb von 48h, die auf die frühere Entnahme folgen, oder bei 48h nach der früheren Entnahme durchgeführt wird.

11. Verfahren nach einem der vorhergehenden Ansprüche, **dadurch gekennzeichnet, dass** die von dem Patienten stammende zu testende Probe eine Plasma- oder Serumprobe ist.

12. Verfahren nach einem der vorhergehenden Ansprüche, **dadurch gekennzeichnet, dass** die Expression von sCD127 auf Proteinebene, vorzugsweise mit Hilfe einer ELISA-Technik gemessen wird.

13. Verfahren nach einem der vorhergehenden Ansprüche, **dadurch gekennzeichnet, dass** die Expression von sCD127 mit Hilfe von Antikörpern Anti-sCD127 und insbesondere von monoklonalen Antikörpern Anti-sCD127 gemessen wird.

14. Verwendung der Messung, *in vitro* oder ex *vivo,* der Expression von sCD127 zur Bestimmung der Anfälligkeit eines Patienten für nosokomiale Infektionen.

15. Verwendung nach Anspruch 14, **dadurch gekennzeichnet, dass** der Patient ein Patient ist, der auf der Intensivstation aufgenommen ist und/oder der einen Stress, wie chirurgischen Eingriff, Verbrennung oder Trauma, der eine systemische Inflammationsreaktion (SIRS) erzeugt, erfahren hat, insbesondere ein Patient in insbesondere schwerem septischem Zustand ist.

16. Verwendung nach Anspruch 15, **dadurch gekennzeichnet, dass** der Patient ein Patient in septischem Schock ist.

17. Verwendung nach Anspruch 15 oder 16, **dadurch gekennzeichnet, dass** die Expression von sCD127 auf Proteinebene, vorzugsweise mit Hilfe einer ELISA-Technik gemessen wird.

18. Verwendung nach einem der Ansprüche 14 bis 17, **dadurch gekennzeichnet, dass** die Expression von sCD127 mit Hilfe von Antikörpern Anti-sCD127, vorzugsweise von monoklonalen Antikörpern Anti-sCD127 gemessen wird.

19. Kit für die Messung, *in vitro* oder ex *vivo,* der Expression von sCD127 in einer biologischen Probe, umfassend:
- spezifische Instrumente oder Reagenzien, die die Messung der Expression von sCD127 in der biologischen Probe ermöglichen, und
- eine positive Kontrollprobe, die eine kalibrierte Probe ist, um die Menge an sCD127 zu enthalten, welche der durchschnittlichen Menge entspricht, die in einem Probenpool von Patienten, von denen man weiß, dass sie eine nosokomiale Infektion entwickelt haben, gemessen wird, und/oder eine negative Kontrollprobe, die eine kalibrierte Probe ist, um die Menge an sCD127 zu enthalten, welche der durchschnittlichen Menge entspricht, die in einem Probenpool von Patienten, von denen man weiß, dass sie keine nosokomiale Infektion entwickelt haben, gemessen wird.

20. Kit nach Anspruch 19, **dadurch gekennzeichnet, dass** die spezifischen Instrumente oder Reagenzien ermöglichen, die Expression von sCD127 zu erfassen und/oder zu quantifizieren, und vorzugsweise auf Proteinebene.

21. Verwendung eines Kits wie er in Anspruch 19 oder 20 definiert ist, zur Bestimmung der Anfälligkeit eines Patienten für nosokomiale Infektionen, wobei der Patient ein Patient ist, der auf der Intensivstation aufgenommen ist und/oder der einen Stress, wie chirurgischen Eingriff, Verbrennung oder Trauma, der eine systemische Inflammationsreaktion (SIRS) erzeugt, erfahren hat.

22. Verwendung eines Kits nach Anspruch 21 zur Bestimmung der Anfälligkeit für nosokomiale Infektionen bei einem Patienten in septischem Schock.

## Claims

1. A process for determining a patient's susceptibility to nosocomial infections, comprising the following steps:
- measuring the expression of sCD127 in a biological sample taken from said patient, or a sample to be tested;
- reaching a conclusion of increased susceptibility to nosocomial infections after comparison of sCD127 expression with a reference value.

2. The process according to claim 1, **characterized in that** the biological sample is from a patient hospitalized in intensive care and/or who has sustained an insult such as surgery, burns, trauma..., generating a systemic inflammatory response or SIRS, in particular taken from a patient with sepsis, in particular severe sepsis.

3. The process according to claim 2, **characterized in that** the biological sample is from a patient with septic shock.

4. The process according to any of claims 1 to 3, **characterized in that** the conclusion is reached of increased susceptibility to nosocomial infections when overexpression of sCD127 is evidenced in the sample to be tested compared with a first reference value and **in that** the first reference value corresponds to the level of sCD127 expression measured in a biological sample taken from a patient hospitalized in intensive care and/or who has sustained an insult such as surgery, burns, trauma, generating a systemic inflammatory response or SIRS, who is known not to have developed a nosocomial infection, in particular a patient with sepsis who is known not to have developed a nosocomial infection, or which corresponds to a mean value of the level of sCD127 expression measured in a pool of samples taken from such patients.

5. The process according to claims 4, **characterized in that** the first reference value corresponds to the level of sCD127 expression measured in a biological sample taken from a patient in septic shock who is known not to have developed a nosocomial infection, or else corresponds to a mean value of the level of sCD127 expression measured in a pool of samples taken from such patients.

6. The process according to any of claims 3 to 5, **characterized in that** measurement of sCD127 expression in the sample to be tested and optionally in the biological sample used to obtain the first reference value is performed within 10 days or at day 10 (D10) after septic shock, preferably within 7 days or at day 7 (D7) after septic shock, more preferably within 4 days or at day 4 (D4) after septic shock and further preferably within 3 days or at day 3 (D3) after septic shock.

7. The process according to any of claims 1 to 3, **characterized in that** the conclusion is reached of increased susceptibility to nosocomial infections when the expression of sCD127 measured in the sample to be tested is not significantly reduced compared with a second reference value, preferably when the expression of sCD127 measured in the sample to be tested is not reduced by more than 25%, preferably not reduced by more than 20% compared with this second reference value and **in that** the second reference value corresponds to the level of sCD127 expression measured in a biological sample from the same said patient taken previously i.e. in a biological sample taken at an earlier time prior to the sample to be tested.

8. The process according to claim 3 or 7, **characterized in that** measurement of sCD127 expression in the sample to be tested is performed at or at about 10 days (D10) after septic shock, preferably at or at about 7 days (D7) after septic shock, more preferably at or at about 4 days (D4) after septic shock.

9. The process according to claim 8, **characterized in that** it is according to claim 7 and **in that** the prior sampling is performed within or at 48h after septic shock and at least 24h before the sample to be tested is taken.

10. The process according to claim 9, **characterized in that** the prior sampling is performed within or at 48h after septic shock, and the sample to be tested is taken within 48h following after the prior sampling or at 48h after the prior sampling.

11. The process according to any of the preceding claims, **characterized in that** the sample to be tested taken from said patient is a plasma or serum sample.

12. The process according to any of the preceding claims, **characterized in that** sCD127 expression is measured at protein level, preferably using an ELISA technique.

13. The process according to any of the preceding claims, **characterized in that** sCD127 expression is measured using anti-sCD127 antibodies, and in particular monoclonal anti-sCD127 antibodies.

14. The use of *in vitro* or *ex vivo* sCD127 measurement to determine a patient's susceptibility to nosocomial infections.

15. The use according to claim 14, **characterized in that** said patient is a patient hospitalized in intensive care and/or having sustained an insult such as surgery, burns, trauma, generating a systemic inflammatory response (SIRS), in particular a patient with sepsis, severe sepsis in particular.

16. The use according to claim 15, **characterized in that** said patient is a patient in septic shock.

17. The use according to claim 15 or 16, **characterized in that** sCD127 expression is measured at protein level, preferably using an ELISA technique.

18. The use according to any of claims 14 to 17, **characterized in that** sCD127 expression is measured using anti-sCD127 antibodies, preferably monoclonal anti-sCD127 antibodies.

19. A kit for *in vitro* or *ex vivo* measurement of sCD127 expression in a biological sample, comprising:
- specific tools or reagents allowing measurement of sCD127 expression in said biological sample; and
- a positive standard sample which is a sample calibrated to contain an amount of sCD127 which corresponds to the mean amount measured in a pool of samples from patients who are known to have developed a nosocomial infection, and/or a negative standard sample which is a sample calibrated to contain the amount of sCD127 which corresponds to the mean amount measured in a pool of samples from patients who are known not to have developed a nosocomial infection.

20. The kit according to claim 19, **characterized in that** said specific tools or reagents allow the detection and/or quantification of sCD127 expression, and preferably at protein level.

21. The use of a kit such as defined in claim 19 or 20 to determine a patient's susceptibility to nosocomial infections, said patient being hospitalized in intensive care and/or having sustained an insult such as surgery, burns, trauma, generating a systemic inflammatory response (SIRS).

22. The use of a kit according to claim 21 to determine the susceptibility to nosocomial infections of a patient with septic shock.
